Europäisches Patentamt

European Patent Office

Office européen des brevets

(19)

(11) Publication number: **0 256 713**
**A2**

(12) **EUROPEAN PATENT APPLICATION**

(21) Application number: 87306750.8

(22) Date of filing: 30.07.87

(51) Int. Cl.⁴: **C12P 21/00** , C07K 15/00 ,
C12N 5/00 , C12N 15/00 ,
A61K 39/40 , G01N 33/569 ,
G01N 33/577 ,
//(C12P21/00,C12R1:91)

---

The applicant has filed a statement in accordance with Rule 28 (4) EPC (issue of a sample only to an expert). Accession number(s) of the deposit(s): ATCC CRL 9477, ATCC HB 9464, ATCC 9149, ATCC HB 9150, ATCC HB 9465, ATCC HB 9161.

(30) Priority: 06.08.86 US 893767
16.07.87 US 71408

(43) Date of publication of application:
24.02.88 Bulletin 88/08

(84) Designated Contracting States:
CH DE FR GB IT LI NL

(71) Applicant: **MERCK & CO. INC.**
**126, East Lincoln Avenue P.O. Box 2000**
**Rahway New Jersey 07065-0900(US)**

(72) Inventor: **Gammon, Maureen**
**397 Charlton Avenue**
**South Orange New Jersey 07097(US)**
Inventor: **Sigal, Nolan H.**
**722 Schackamaxon Drive**
**Westfield New Jersey 07090(US)**
Inventor: **Zweerink, Hans J.**
**42 Beechwood Drive**
**Shrewsbury New Jersey 07701(US)**
Inventor: **Miner, Karen M.**
**219 Phelps Road**
**Ridgewood New Jersey 07450(US)**

(74) Representative: **Hesketh, Alan, Dr.**
**European Patent Department Merck & Co.,**
**Inc. Terlings Park Eastwick Road**
**Harlow Essex, CM20 2QR(GB)**

(54) **Therapeutic human antipseudomonas aeruginosa antibodies.**

(57) Human lymphoblastoid cell lines and hybridomas are developed which secrete antibodies specifically reactive with lipopolysaccharide that is present on the surface of Pseudomonas aeruginosa. The individual monoclonal antibodies are immunospecific for one or more of the Fisher immunotypes of P. aeruginosa. The anti-Pseudomonas monoclonal antibodies are used individually or in combination to therapeutically or prophylactically treat P. aeruginosa infections. One or more of the monoclonal antibodies are co-administered with one or more antibiotics to therapeutically or prophylactically treat immunocompromised and immunosuppressed individuals.

EP 0 256 713 A2

## THERAPEUTIC HUMAN ANTI-PSEUDOMONAS AERUGINOSA ANTIBODIES

### BACKGROUND OF THE INVENTION

Pseudomonas aeruginosais a ubiquitous bacterium that frequently infects compromised hosts, especially patients with cystic fibrosis, thermal burns, cancer patients undergoing radiation treatment or chemotherapy, patients undergoing abdominal surgery and patients in intensive care units. Nosocomial infections due to P. aeruginosa are difficult to treat because the causative strains are often resistant to antibiotics. The lack of effective antibiotic therapy has stimulated a search for additional strategies to treat and prevent Pseudomonas infections. The possibility of immunologic intervention of P. aeruginosa infection began with the detection of anti-Pseudomonas aeruginosa antibodies in normal human serum (Young, et al. Infect. Immun. 2: 495-503 (1970)). The presence of polyclonal antibodies reactive with Pseudomonas aeruginosa exotoxin A and lipopolysaccharide (LPS) in patients with P. aeruginosa septicemia correlated with increased survival, Pollack and Young, J. Clin. Invest. 63: 276-286 (1979). It has recently been demonstrated in a murine burn wound sepsis model that antilipopolysaccharide immunoglobulin could protect against P. aeruginosa infection, Cryz, et al. Infec. Immun. 39:1072-1079 (1983). Human polyclonal IgG containing antibodies reactive with P. aeruginosa immunotypes 1, 2, 4 and 6, International Antigenic Typing System Serotypes (IATS), protected mice from challenge with viable P. aeruginosa of the same immunotype but did not protect against other immunotypes, Collins and Roby, Am. J. Med. 76 : 168-174, 1984. Murine monoclonal antibodies (MAb) have been prepared that react with Homma serotypes, Homma et al., JPN J. Exp. Med. 41: 89-94 (1971), 2, 7, and 13, P. aeruginosa LPS. Thes MAbs were highly protective against infection in a mouse system, Sawada, et al. J. Infec. Dis. 150:570-576, (1984).

The therapeutic use in humans of either human derived polyclonal or murine monoclonal anti-P. aeruginosa antibodies has severe limitations. It is very difficult to prepare sufficient quantities of the appropriate polyclonal antibodies from human donor sera for widespread therapeutic use. The use of murine MAbs, reactive with P. aeruginosa, is not practicable in humans since the antibodies would be recognized as foreign and the individual would produce a potentially detrimental immune response against the therapeutic MAbs.

Human monoclonal antibodies have been produced which specifically react with P. aeruginosa exotoxin, European Patent Application 176 365. This antibody was capable of protecting mice when challenged with P. aeruginosa organisms. Human monoclonal antibodies capable of agglutinating P. aeruginosa cells have recently been described, Japanese Patent Application 60248626. These antibodies are of the IgG isotype and it is suggested that they may protect mice against P. aeruginosa infection.

### SUMMARY OF THE INVENTION

Human lymphoblastoid cell lines and hybridomas are developed which secrete antibodies specifically reactive with lipopolysaccharide that is present on the surface of Pseudomonas aeruginosa. The individual monoclonal antibodies are immunospecific for one or more of the Fisher immunotypes of P. aeruginosa. The anti-Pseudomonas monoclonal antibodies are used individually or in combination to therapeutically or prophylactically treat P. aeruginosa infections. One or more of the monoclonal antibodies are co-administered with one or more antibiotics to therapeutically or prophylactically treat immunocompromised and immunosuppressed individuals.

### OBJECTS OF THE INVENTION

It is, accordingly, an object of the present invention to provide monoclonal antibodies that will passively protect against Pseudomonas aeruginosa infection. Another object is to provide a means for obtaining these monoclonal antibodies. A further object is to produce hybridoma cell lines capable of secreting monoclonal anti-Pseudomonas aeruginosa antibodies. Still another object is to provide pharmaceutical compositions comprising anti-P. aeruginosa monoclonal antibodies. Another object is to provide methods of treatment comprising administering anti P. aeruginosa monoclonal antibodies when a therapeutic or prophylactic treatment for P. aeruginosa infection is indicated. Another object is to provide methods of treatment comprising the co-administration of anti-P. aeruginosa monoclonal antibodies and antibiotics when a therapeutic or porphylactic treatment for a P. aeruginosa infection and mixed microbial infections including P. aeruginosa and one or more other microorganism is indicated. Another object is to provide monoclonal antibodies for the identification of P. aeruginosa immunotypes and serotypes. These and other objects of the present invention will be apparent from the following description.

## DETAILED DESCRIPTION

The present invention relates to the production of monoclonal antibodies (MAb) specifically reactive with defined immunotypes of Pseudomonas aeruginosa and the cell lines that produce the specific monoclonal antibodies. The term monoclonal antibody, as used herein, refers to a single antibody species with homogenous binding characteristics for the relevant antigen. Homogenous binding as used herein refers to the ability of the antibody species to bind to a specific antigen or epitope, such as a specific carbohydrate sequence on the Pseudomonas aeruginosa lipopolysaccharide (LPS). The monoclonal antibodies are produced by and secreted from either lymphoblastoid cells or hybridomas. Lymphoblastoid cells are antibody producing B lymphocytes that have been transformed with the Epstein-Barr virus (EBV) which enables the cells to grow for extended periods of time and for numerous generations invivo, and to produce significant levels of the specific antibody. Hybridoma, as used herein, refers to cells formed by the fusion of a non-EBV-transformed antibody producing B lymphocyte or EBV-transformed antibody producing B lymphoblastoid cell with an immortalizing cell to form a hybrid cell, with EBV transformed B lympoblastoid cells being preferred. Antibody producing B lymphocytes or lymphoblastoid cells can be obtained by immunizing a host and isolating antibody producing cells or screening normal individuals for the presence of specific antibody producing cells. The cell lines may be cloned to select for cells that produce a single antibody with homogenous binding characteristics. Selective cloning may take place prior to fusion, wherein the lymphoblastoid cell is cloned, or cloning may take place following fusion, with the hybridoma cell being cloned. Cloning procedures are known in the art. Both lymphoblastoid cell lines and hybridoma cell lines can be used to produce monoclonal antibodies.

While the following description and examples illustrate the present invention with respect to monoclonal antibodies reactive with P. aeruginosa immunotypes 1, 2, 3, 4, 5, 6 and 7, Fisher et. al., J. Bacteriol 98: 835-836 (1969), it is understood that the present invention is applicable to monoclonal antibodies reactive with other P. aeruginosa immunotypes and serotypes. Other serotypes include, but are not limited to, IATS 3 and 15.

Peripheral blood lymphocytes are obtained from healthy human volunteers or children with cystic fibrosis by the separation of whole blood using discontinuous density grandient centrifugation. The purified lymphocytes are mixed with the supernatant fluid from cells persistently infected with and releasing Epstein-Barr virus (EBV), preferably B95-8 cells. To obtain the EBV rich supernatant fluid B95-8 cells are cultured at a concentration of about $1 \times 10^5$ cells/ml to about $3 \times 10^5$ cells/ml, with about $2 \times 10^5$ cells/ml being preferred. The cells are cultured at about 37°C in an acceptable media, preferably RPMI-1640, containing about 10% fetal bovine serum plus antibiotics and glutamine for about 7 days. The cell free culture fluid is obtained by centrifugation and filtration through a 0.45 mm filter. After incubation of lymphocytes and EBV for about 2 hours the cells are pelleted by centrifugation, resuspended in growth medium, preferably RPMI 1640, supplemented with about 15% fetal bovine serum, glutamine and antibiotics and either grown in mass culture or more preferably distributed into culture plates at concentrations of from about 500 cells per well to about 7,000 cells per well. During this incubation period the EBV infects the lymphocytes, including the B lymphocytes which are producing specific antibody, Bird et al., J. Exp. 154: 832-839 (1981). Viral infection results in transformation of the cells with the lymphochtes being termed lymphoblastoid cells and the B lymphocytes termed B lymphoblastoid cells. B lymphoblastoid cells can grow for extended periods of time and secrete economically significant levels of specific antibodies with homogenous binding characteristics. Economically significant refers to levels that can be produced in vitro which will allow the quantities necessary for therapeutic treatment. Growth of the lyphoblastoid cells is enhanced by the addition of about $5 \times 10^4$ to about $5 \times 10^6$, with $5 \times 10^5$ preferred, per well of irradiated human cord blood lymphocytes stimulated with phytohemagglutinin, after purification by discontinuous density gradient centrifugation or supernatant fluids from growing cells, with irradiated cells being preferred as described by Stein and Sigal, Cell Immun. 79: 309-319 (1983). To assay for the production of antibodies against each of the seven Fisher P. aeruginosa immunotypes the culture fluid is collected at about 3 and about 4 weeks after initial plating and tested by solid phase radioimmunoassays (SPIRA), enzyme linked immunoassay or fluorescent immunoassay, with SPIRA being preferred.

Screening of cultures established from lymphocytes from adult human donors identified three cell lines which produce antibodies against P. aeruginosa Fisher immunotypes 2, 4 and 5 respectively. The cell line producing anti-immunotype 2 antibody is designated RM5 and the resulting immunoglobulin is termed MAb-RM5. The cell line secreting anti-P.aeruginosa immunotype 5 antibody is designated 11F9 and the resulting immunoglobulin is termed MAb-11F9. The cell line secreting anti-P. aeruginosa immunotype 4 antibody is designated FDD7 and the resulting immunoglobulin is termed MAb-FDD7. The FDD7 cells are subjected to the limiting dilution procedure of Kozbor et al., J. Immunol. 133: 3001-3005 (1984) and an antibody producing clone isolated. The antibody is identical to MAb-FDD7 and is termed MAb-FDD7.7 and the cloned lymphoblastoid cell line is termed FDD7.7. Transformation of lymphocytes from patients with cystic fibrosis cells resulted in a cell line which produces antibodies reactive with P. aeruginosa Fisher immunotypes 3, 6 and 7. This cell line is designated CF-9H10 and the resulting immunoglobulin is termed MAb-9H10. Characterization of antibodies reveals that MAb-RM5 reacts with lipopolysaccharide (LPS) extracted from P. aeruginosa Fisher immunotype 2 and exhibits an IgM isotype with $x$ (kappa) light chains. Cell line 11F9 secretes MAb-11F9 antibody with an IgM isotype and reacts with LPS extracted from P. aeruginosa Fisher immunotype 5. The MAb-FDD7.7 secreted by the cell line FDD7.7 exhibits an IgM isotype, has $x$ light chains and reacts with LPS extracted from P. aeruginosa Fisher immunotype 4. Cell line CF-9H10 secretes MAb-9H10 antibody with an IgM isotype and $x$ light chains and reacts with LPS extracted from P. aeruginosa Fisher immunotypes 3, 6 and 7. The antibody isotypes are determined by a SPIRA assay in which assay wells are coated with goat antibodies against the heavy chain of human immunoglobulin M,G, or A. The wells are blocked and the culture fluids are added and incubated for several hours. After extensive washing [125]-iodinated goat antibody specific for human $x$ or $\lambda$ light immunoglobulin chain was added and binding determined. The results showed that MAb-FDD7.7, MAb-RM5, MAb-9H10 and MAb-11F9 contain $\mu$ heavy chains while MAb-FDD7N7, MAb-RM5 and MAb-9H10 contain $x$ light chains.

The cell lines RM5, FDD7.7, CF-9H10 and 11F9 secrete into the culture fluid antibodies that induce a high degree of passive protection when administered to immunodeficient animals, preferably mice. Antibody produced by the RM5 cell line specifically protects immune deficient (Xid) mice against challenge with viable P. aeruginosa Fisher immunotype 2 in dosages of from about 0.02 $\mu$g per mouse to about 2 $\mu$g or greater per mouse when administered about 2 hours prior to challenge to about 2 hours or more after challenge. Under similar conditions MAb-FDD7.7 is able to specifically protect mice from challenge with P. aeruginosa Fisher immunotype 4. Administration of similar dosages of either MAb-9H10 or MAb-11F9 protect Xid mice from challenge with viable P. aeruginosa Fisher immunotypes 3, 6 and 7 or immunotype 5.

Lymphoblastoid cells, isolated as described above, are the critical stage in the preparation of anti-P. aeruginosa monooclonal antibody cell lines and consequently the production and isolation of therapeutically effective specific monoclonal antibodies. Once a specific antibody producing lymphoblastoid cell is identified and isolated it is cultured in vitro and the corresponding monoclonal antibody collected as described above. While the EBV transformed lymphoblastoid cell lines generally produce adequate levels of monoclonal antibodies some may have a limited life span and may generally fail to grow for periods longer than about 2 to about 14 months depending on the specific cell line. It should be noted, however, that most anti-P. aeruginosa lymphoblastoid cell lines continue to secrete antibody and replicate for several months after isolation with some producing therapeutically sufficient amounts of monoclonal antibody for periods longer than a year. Some lymphoblastoid cell lines cannot be cultured for extended periods of time and must fused with immortalizing cells. Immortalizing cells are defined as cells which are capable of continued replication for indefinite periods of time and when fused with lymphoblastoid cells will impart this longevity to the resulting cell type, termed a hybridoma. An acceptable immortalizing cell fusion partner will contribute immortalizing properties without altering the specific antibody produced by the lymphoblastoid fusion partner. Hybridomas resulting form the fusion of a specific lymphoblastoid cell with an immortalizing cell, termed a primary fusion, can be fused with a second immortalizing cell, a secondary fusion, to produce hybridomas with the immortalizing characteristics of the second fusion partner and the antibody characteristics of the lymphoblastoid cell. In either a primary fusion or secondary fusion the fused cells are screened for the production of the specific monoclonal antibody characteristic of the lymphoblastoid cell by techniques known in the art, see Kohler and Milstein, Nature 256: 495-497 (1975) generally and Kaplan, U.S. Patent No. 4,582,797 for multiple fusions. Consequently, all cells derived from the original monoclonal antibody producing lymphoblastoid cell produce exactly the same antibody and are termed derivative cells. Derivative cells may include any progeny, descendants or issue of the lymphoblastoid cells, any fusion derived cells including hybridomas and the progeny, descendants or issue thereof regardless of generation or karyotypic identity. Immortalizing cells may include intra alia, myelomatous B lymphocytes, non-antibody producing B lymphoblastoid cells, hetermyelomas derived from interspecies fusions or other transformed cells isolated from various mammalian species including human, mouse, rat and guinea pig, with human lymphoblastoid and human mouse hetermyelomas being preferred. Acceptable fusion partners include, but are not limited to human myeloma cell GM4672, Shonefeld et al . J. Clin. Invest. 70: 205-208 (1982); human lymphoblastoid KR-4 and human hybridmyeloma KR-12, Kozbor et al., J. Immunol. Methods 81: 31-42 (1985); human/mouse heteromyeloma line SHM-D33, Teng et al., Proc. Nat. Acad. Sci. USA 80: 7308-7312 (1983), see also U.S. Patent 4,574,116, and SBC-H2O, Foung et al. J. Immunol. Meth. 70: 83-90 (1984); human lymphoblastoid WI-L2-729 HF$_2$, U.S. Patent 4,594,325; or mouse myeloma cells such as NS. 1, Cote et al. Proc. Natl. Acad. Sci. USA 80: 2026-2030 (1983), with KR-4 and SHM-D33 being preferred and the SHM-D33 being the most preferred. The RM5, FDD7.7, CF-9H10 and 11F9 cells are fused with either KR-4 cells described by Kozbor et al. Proc. Natl. Acad. Sci. USA 79 6651-6655 (1982) or SHM-D33 cells as described in U.S. Patent No. 4,582,797. About $10^7$ antibody producing lymphoblastoid cells, RM5, FDD7.7, CF-9H10, or 11F9 and about $10^7$ KR-4 cells or SHM-D33 cells are mixed in the presence of about 45% polyethylene glycol, about 4000 mol. wt., for about 1 minute for the primary fusion. Next the cell suspension is diluted to about 1:10 over about a 10 minute period and incubated at about 37°C for about 30 minutes. The polyethylene glycol is removed by centrifugation and the cells plated in culture medium, preferably RPMI 1640 supplemental with about 15% fetal calf serum, to a concentration of about $10^5$ cells per well. Each well receives about $5 \times 10^4$ irradiated human cord blood lymphocytes per well. After incubation at about 37°C for about 24 hours the culture medium is removed and replaced with fresh RPMI-1640 containing hypoxanthine, about $10^{-4}$M, aminopterin, about $4 \times 10^{-7}$M, thymidene, about $10^{-5}$M and oubain, about $5 \times 10^{-4}$M. The culture medium is changed about every 3-4 days. After about 3 weeks the culture fluid is assayed for P. aeruginosa Fisher immunotype specific antibodies as described above. It should be kept in mind, however, that any of the lymphoblastoid cells described above can be fused with any acceptable immortalizing cell, screened and a specific hybridoma isolated. The criteria for screening and isolation is that the cell produces specific immunotype antibody or antibodies which react with one or more of the Fisher immunotypes. As stated previously these hybidomas can be fused one or more times with other immortalizing cells. Further fusions are screened in the same manner as that for lymphoblastoid and primary fusion cells. The procedures are contained herein or are known in the art. Primary fusion with anti-P. aeruginosa producing lymphoblastoid cells may result in the following hybrids or hybridomas: RM5 × KR-4 or SHM-D33; FDD7.7 × KR-4 or SHM-D33, CF-9H10 × KR-4 or SHM-33; 11F9 × KR-4 or SHM-D33. Characteristic hybridomas include, but are not limited to, RK-52, RM5 × KR-4, FK-16-4, FDD7.7 × KR-4; CK-1G11-18, CF1219-9H10 × KR-4, RS-1H7, RM5 × SHM-D33; RS-1G11, RM5 × SHM-D33. The hybridomas produced by primary fusion are cloned by the limiting dilution technique of Kozbor et al., J. Immunol. 133: 3001-3005 (1984) if the lymphoblastoid cells were not cloned prior to fusion. Secondary fusions are carried out employing the techniques just described with the resulting hybridomas cloned by the Kozbor procedure. The P. aeruginosa specific MAbs produced by the individual hybridomas have all the characteristics of the specific monoclonal antibody produced by the corresponding lymphoblastoid cell line as defined by antigen recognition and mouse protection. The RM5 hybridomas produce monoclonal antibody which specifically protects mice from challenge with Fisher immunotype 4 P. aeruginosa. The CF9H10 derived hybridomas produce monoclonal antibody which specifically protects mice from challenge with P. aeruginosa Fisher immunotype 2 while the FDD7.7 hybridomas produce monoclonal antibody which specifically protects mice form challenge with P. aeruginosa Fisher immunotypes 3, 6 and 7 while the 11F9 derived hybridomas produce monoclonal antibody which specifically protects mice from challenge with Fisher immunotype 5.

The therapeutic and prophylactic use of monoclonal antibodies against P. aeruginosa includes one or more of the antibodies reactive against the various Fisher immunotypes, 1-7 or IATS serotypes such as 3 and 15. The broadest therapeutic and prophylactic efficacy will be obtained by including monoclonal antibodies reactive with all seven of the Pseudomonas immunotypes and the two serotypes. This may include seven or more individual monoclonal antibodies with a single immunotype or serotype specificity or fewer antibodies if they contain multiple immunotype specificities. It is generally assumed that a prophylactic and therapeutic preparation of anti-P. aeruginosa monoclonal antibodies will contain at least two separate monoclonals.

The monoclonal antibodies of the present invention can administered to mammals, including humans, either alone, or preferably in combination with pharmaceutically acceptable diluents or carriers, in a pharmaceutical composition, according to standard pharmaceutical practice. Acceptable diluents include, but are not limited to, physiologic saline, buffered saline, Ringer's injection, dextrose injection, dextrose saline and lactated Ringer's injection or diluent solutions containing additional therapeutic agents such an antibiotics or other monoclonal antibodies of therapeutic value. Other monoclonal antibodies of therapeutic value include specific antibodies reactive against other gram negative or gram positive bacteria and other microorganisms which are or may develop resistance to antibiotics. The anti-P. aeruginosa monoclonal antibodies can be administered locally or parenterally. Local administration includes topically, on the skin, and by inhalation into the lungs. Topical pharmaceutical carriers, may include but are not limited to, ointments, pastes, solutions, gels, solid water soluble polymers such as albumins, gelatins, hydroxypropyl cellulose, pluronics, tetronics or alginates. Parenteral administration includes intravenous, intramuscular, intraperitoneal and subcutaneous administration. For parenteral administration, sterile solutions of the monoclonal antibodies are usually prepared, and the pH of the solutions are suitably adjusted and buffered. The total concentration of solutes should be controlled to render the preparation isotonic.

When monoclonal antibodies against P. aeruginosa are used therapeutically and prophylactically in a human subject, the daily dosage will normally be determined by the prescribing physician. Moreover, the dosage will vary according to the age, weight and response of the individual patient, as well as the severity of the patient's symptoms. However, in most instances, an effective daily dosage will be in the range from about 1 μg per kg body weight to about 300 μg per kg body weight in a single or divided doses. On the other hand, it may be necessary to use dosages outside these limits in some cases.

The scope of the present invention also includes the combined therapeutic and prophylactic use of anti-P. aeruginosa monoclonal antibodies combined with antimicrobial agents such as antibiotics. Antibiotics are chemical substances produced by various species of microorganisms that suppress the growth of other microorganisms and may eventually destroy them. This combined or co-administrative therapy is advantages for treating both P. aeruginosa infections and mixed infections caused by P. aeruginosa plus one or more bacteria or other microorganisms. In the first instance the action of the monoclonal antibodies against P. aeruginosa may be enhanced by the presence of an antibiotic which can suppress growth of the bacteria and allow the monoclonal antibodies to attach and initiate immunological clearance of the Pseudomonads . The converse may also occur, that is the monoclonal antibodies may attach to the bacterial cell and initiate immune clearance which can enhance antimicrobial activity of the antibiotic. In either case the combined thereapy may be more effective than either the monoclonal antibodies or the antibiotic alone. In the combined therapy the amount of each component must be adjusted so that the total will have the greatest antimicrobial effect. This may entail either increasing or decreasing the amount of each component, monoclonal antibodies and antibiotics, to arrive at the optimal dose. This can most easily be accomplished by the attending physician.

Mixed infections may generally occur in individuals suffering from various forms of immunological insufficiency or defficiency such as thermal burn patients, cancer patients undergoing radiation treatment or chemotherapy, patients undergoing abdominal surgery and patients in intensive care units. The anti-P. aeruginosa monoclonal antibodies may induce immune clearance of Pseudomonas cells while the antibiotics destroy or suppress the other bacteria. The antibiotics may also enhance the activity of the monoclonal antibodies as described above. Monoclonal antibodies reactive with P. aeruginosa may also be administered with antibiotics when the actual cause of infection is not known. The use of a broad spectrum antibiotic or more than one antibiotic given along with anti-P. aeruginosa monoclonal antibodies would enhance the spectra of antibacterial activity in patients especially if the infective agents, such as bacteria, have not been identified. It is thus, advantageous to select the appropriate antibiotics so that there is sufficient breadth of activity to control the infectious microorganisms. The effective dosages of each component will be determined as described above. The antibiotics may be selected from, but are not limited to, the following groups, of which examples are given, without being exhaustive:

1. Aminoglycosides

amikacin     lincomycin
clindamycin     netilmicin sulfate
dibekacin     tobramycin
gentamicin sulfate

2. Cephalosporins

cefaclor     cefotiam
cefadroxil     cefoxitin
cefamandole sodium     cefpiramicle sodium

cefazolin    cefpiramide sodium
cefbuperazone sodium    cefsueodin sodium
cefoperazone    ceftriaxone sodium
cefoperazone sodium    ceftzxidime
cefotaxime    ceftizoxime
ceftaxime sodium    cephalexin
cafotetan    oxacephalosporin
cefmetazole    ceftriaxone

cefmeoxime    cefoperazone
ceftazidime    cefuroxime
cephapuin sodium    latamoxef

3. Macrolids

erythromycin    erythromycin ethyl succinate

4. Penecillins

amoxycillin    penecilin G benzathine
amoxicillin    penecillin G sodium
ampicillin    penecillin V
mezlocillin    penecillin V potassium
penecillin G    piperacillin piperacillin sodium

5. Tetracyclines

minocyline hydrochloride
tetracycline hydrochloride
doxycycline

6. Polypeptides    polymyxin G    bacitracin

7. Polyenes    amphotericin B    nystatin
of injectible and topical antibiotics.

When the monoclonal antibodies, one or more, are co-administered with antibiotics, one more, the dairy dosage of antibiotics will normally be determined by the prescribing physician as recommended by the antibiotic manufacturer or normal medical practice. Moreover, the dosage will vary according to the age, weight and response of the individual patient, as well as the severity of the patient's symptoms. Dosages for the antibiotics are known in the art.

The following Examples illustrate the present invention without, however, limiting the same thereto.

EXAMPLE 1

7

## TRANSFORMATION AND IDENTIFICATION OF SPECIFIC ANTIBODY PRODUCING CELLS

Human peripheral blood lymphocytes from normal volunteers or cystic fibrosis patients were obtained from freshly drawn peripheral blood by Hypaque-Ficol density gradient centrifugation at $250 \times$ g. Lymphocytes, $1 \times 10^7$, isolated from 10 ml of heparinized blood were suspended in 1 ml RPMI-1640 and after the addition of 3 ml of cell free culture fluid obtained from B95-8 cells which continuously produce and release infectious Epstein-Barr virus, Rosen et al. J. Immunol. 130:2899-2902 (1983) they were incubated for 2 hours at 37°C. The lymphocytes were collected by centrifugation, resuspended in culture medium, RPMI-1640 supplemented with 15% fetal bovine serum, glutamine and antibiotics and distributed in 96 well culture plates at concentration of $1 \times 10^3$, $3 \times 10^3$ or $6 \times 10^3$ cells per well in 0.2 ml of supplemental RPMI-1640 culture fluid. Growth of the transformed cells was enhanced by the addition of Hypaque-Ficol purified, irradiated, 2500 rads, human cord blood lymphocytes at a concentration of $5 \times 10^5$ cells per well stimulated by the addition of phytohemagglutinin (Difco), 1:1000, Stein and Sigal, Cell Immunol. 79: 309-319 (1983). The cells were incubated at 37°C in a humidified atmosphere of 5% $CO_2$-95% air. The lymphocyte cultures were fed every seventh day for the duration of the initial isolation period.

In order to identify cells that produce antibody against P. aeruginosa, the culture fluid from individual wells was collected at weeks 3 and 4 after plating and assayed for the presence of antibodies. The presence of P. aeruginosa LPS immunotype or serotype antibodies was determined by solid phase radioimmunoassay (SPIRA). Antigen for the SPIRA assay was prepared by growing P. aeruginosa immunotypes 1 through 7 in tryptic soy broth to a concentration of approximately $10^9$ bacteria per ml. The bacterial cells were harvested by centrifugation, 5,000 rpm for 20 minutes, washed twice with phosphate buffered saline, 0.15 M NaCl, 0.01 M phosphate buffer, pH 7.2 (PBS), resuspended in PBS at a concentration to yield 12-16% transmission and placed in a boiling water bath for 2.5 hours after which thimerosal was added to 0.01 percent. For the SPIRA each bacterial immunotype was diluted 10-fold in Tris, 0.02 M, pH 9.0, and 0.1 ml was added to the appropriate wells of 96 well round-bottom polyvinyl assay plates. Attachment of the Pseudomonas immunotypes was carried out at 4°C for a minimum of 16 hours. The supernatant fluid was removed and the wells blocked with PBS plus 0.02% sodium azide (PBS-AZ) containing 1% bovine serum albumin. Following blocking for 1 hour at 25°C the wells were washed with PBS-AZ and 25 µl of culture supernatant fluid from the transformed cells was added. After incubation at room temperature for at least 3 hours, the wells were washed with PBS to remove any free antibody and 100 µl $^{125}$Iodine labeled, affinity purified goat antibodies specific for human λ light chain and x light chain, diluted in 1% BSA PBS-AZ were added. Concentrations of both antibodies were adjusted such that 25,000 cpm of each were added per well. The plates were incubated overnight at 4°C, washed with PBS-AZ and each well removed for determination of radioactivity. Cell free lipopolysaccharide prepared by the method of Westphal and Jann, Methods in Carbohydrate Chemistry V, 83-91, 1965, is substituted for whole bacterial cells in the SPIRA assay. Nucleic acids in the aqueous phase were removed by treatment with DNAase adn RNAase (20 µg/ml each) at 37°C for 2 hours and residual proteins were removed by treatment with 20 µg/ml pronase for 16 hours. LPS was purified centrifugation at $100,000 \times$ g for 16 hours, Johnson and Perry, Can. J. Microbiol. 22: 29-34 (1976). Purified LPS was suspended at 1 mg/ml in water containing 1 percent triethylamine. Prior to the SPIRA it was diluted 100-fold in Tris, 0.02M, pH 9.0.

Culture fluid devoid of antibodies to any of the immunotypes produced less than 200 counts per minute (cpm) per well and such values were considered background. Culture fluid supernatants that generated counts at least 2-3 times higher than background were considered antibody positive.

At least 20 EBV transformed samples were tested, nearly 16,000 culture fluids, against all seven P. aeruginosa immunotypes before a single culture fluid showed a positive response to Fisher immunotype 2. These cells, isolated from a healthy volunteer, secreted immunotype 2 specific monoclonal antibody and were designated RM5 and the monoclonal antibody was designated MAb-RM5.

Under similar experimental conditions a second well was identified, which contained cells that secreted immunotype 4 specific immunoglobulin. This cell line, also derived from a healthy volunteer, was designated FDD7 and its monoclonal antibody was designated MAb-FDD7. The FDD7 cells were subjected to the limiting dilution procedure of Kozbor et al., J. Immunol. 133: 3001-3005 (1984) and an antibody producing clone isolated. The antibody is identical to MAb-FDD7 and is termed MAb-FDD7.7 and the cloned hybridoma is termed FDD7.7.

After testing under similar conditions, 10 cystic fibrosis lymphocyte transformations, a well was identified containing cells that secreted immunotype 3,6 and 7 specific immunoglobulin. This cell line was designated CF-9H10 and its monoclonal antibody was designated MAb-9H10. A sample of the lymphoblastoid cell line CF-9H10 was deposited in the American Type Culture Collection, 12301 Parklawn Drive, Rockville Maryland 20852, USA, on July 9, 1987, under The Budapest Treaty and has been assigned ATCC number CRL 9477.

After testing under similar conditions, 80 normal lymphocyte transformations, approximately 64,000 wells, a well was identified containing cells that secreted immunotype 5 specific monoclonal antibody. This cell line was designated 11F9 and its monoclonal antibody was designated MAb-11F9. A sample of the lymphoblastoid cell line 11F9 was deposited in the American Type Culture Collection 12301 Parklawn Drive, Rockville, Maryland 20852, USA, on July 1, 1987 under The Budapest Treaty and has been assigned ATCC number HB 9464.

## EXAMPLE 2

### CHARACTERIZATION OF MONOCLONAL ANTIBODIES

Antibodies MAb-RM5, MAb-FDD7.7, MAb-9H10 and MAb-11F9 from Example 1 were assayed using a SPIRA test to determine the specificity to lipopolysaccharide (LPS) extracted from P. aeruginosa of the various immunotypes. MAb-RM5 reacted only with LPS from P. aeruginosa immunotype 2 while MAb-FDD.7 bound specifically to LPS from P. aeruginosa immunotype 4. MAb-9H10 reacted only with LPS from P. aeruginosa immunotypes 3, 6 and 7 while MAb-11F98 bound specifically to LPS immunotype 5. The interaction between MAb-9H10 and LPS from P. aeruginosa immunotypes 3, 6 and 7 was investigated further by electrophoretic and immunochemical analyses. The procedures were similar to those described by Sadoff et al., Antibiot. Chemother. 36: 134-146 (1985). Briefly, each LPS preparation was subjected to electrophoresis in a polyacrylamide gel, followed by transfer to nitro-cellulose paper strips. These strips were incubated with MAb-9H10 and the binding of antibody was visualized with 125-iodine labeled goat immunoglobulin against human IgM followed by autoradiography. For the LPS from all three immunotypes (3, 6 and 7) it was found that MAb-9H10 bound to molecular species with electrophoretic migration patterns that were typical and characteristic for LPS. These experiments established clearly that MAb-9H10 reacts with LPS and not with another molecular species that could be present as a contaminant in the LPS preparations. A SPIRA assay procedure was used to determine the isotype of the monoclonal antibody heavy and light chains. Microassay wells were coated with goat antibodies against human immunoglobulin M, G, or A. After blocking, tissue culture supernatants containing MAb-FDD7.7, MAb-RM5, MAb-9H10 or MAb-11F9 were added for several hours. After extensive washing of the wells 125-iodinated goat antibody specific for the human $x$ or $\lambda$ light immunoglobulin chain or buffer was added and binding determined as described before. A positive reaction for MAb-FDD7.7, MAb-RM5, MAb-9H10 and MAb-11F9 was observed in wells coated with goat anti-human IgM while MAb-FDD7.7, MAb-RM5 and MAb-9H10 reacted with 125-iodine labeled goat anti-human $x$ light chain. This indicates that the MAb-FDD7.7, MAb-RM5, MAb-9H10 and MAb-11F9 antibodies contain a $\mu$ heavy chain while the MAb-FDD7.7, MAb-RM5 and MAb-9H10 antibodies contain $x$ light chain.

## EXAMPLE 3

### PASSIVE PROTECTION WITH MONOCLONAL ANTIBODIES DERIVED FROM LYMPHOBLASTOID CELLS

The monoclonal antibodies from Example 1 were assayed for their ability to protect mice against challenge with viable P. aeruginosa. Immune-deficient (Xid) male mice derived from a cross between female CBA/N and male DBA/2 or C57B1/6 were made neutropenic by treatment 4 days prior to challenge with cyclophosphamide, 250 mg per Kg body weight. The MAb raised against the specific immunotype was administered as cell supernatant fluid, for this purpose cells were grown in the absence of antibiotics, either intraperitoneally (i.p.) or intravenously (i.v.). The antibody was administered at 2 hours (-2) or 15 minutes (-0.25) prior to bacterial challenge or 2 hours (+2) after challenge with the corresponding immunotype. The appropriate MAb was given as a single dose, 1 μg IgM per 0.5 ml, with controls receiving culture medium similar to that used to grow the antibody producing hybridoma cells, RPMI-1640 plus 15% fetal bovine serum. The efficacy of the MAb was determined by calculating the number of viable bacteria required to kill 50% of the experimental animals in a group (LD₅₀). LD₅₀ values were determined as described by Reed and Muench, Am. J. Hygiene 27: 493-497 (1985). Specificity was evaluated by administering MAb-RM5 and challenging with virulent P. aeruginosa of immunotype 4. The following results were obtained.

## PROTECTION WITH MAb-RM5

| Treatment | Route of Injection | Time Hours | Challenge Immunotype | $LD_{50}$ |
|---|---|---|---|---|
| Control | I.V. | -2 | 2 | 1 |
| MAb-RM5 | I.V. | -2 | 2 | $1.4 \times 10^4$ |
| MAb-RM5 | I.V. | -0.25 | 2 | $6.7 \times 10^3$ |
| Control | I.P. | -2 | 2 | $2.1 \times 10^1$ |
| MAb-RM5 | I.P. | -2 | 2 | $4.7 \times 10^3$ |
| MAb-RM5 | I.P. | -0.25 | 2 | $2.1 \times 10^3$ |
| Control | I.P. | -2,+2 | 4 | 4.6 |
| MAb-RM5 | I.P. | -2,+2 | 4 | $3.1 \times 10$ |

These results show that immunotype specific MAb protects mice against challenge with the corresponding P. aeruginosa. Monoclonal antibody against immunotype 2, MAb-RM5, did not protect against challenge with P. aeruginosa immunotype 4.

Effective dosage levels were determined by injecting Xid mice with various concentrations of MAb-RM5 2 hours prior to challenge with the appropriate P. aeruginosa immunotype. Each animal received intravenously, 0.5 ml culture fluid containing the desired concentration of MAb. The following results were obtained.

## DOSE RESPONSE

| Concentration µg/dose | $LD_{50}$ |
|---|---|
| -- | 3.15 |
| 0.002 | $1.50 \times 10^1$ |
| 0.02 | $4.20 \times 10^2$ |
| 0.2 | $3.40 \times 10^4$ |
| 2.0 | $6.40 \times 10^4$ |

These results show that MAb levels as low as 0.02 µg per mouse will protect mice from challenge with the corresponding P. aeruginosa immunotype.

MAb-FDD7.7 was effective, when given I.V. 2 hours prior to challenge, in protecting Xid mice against challenge with P. aeruginosa immunotype 4. The following results were obtained.

PROTECTION WITH MAb-FDD7.7

| Treatment | Concentration µg/dose | $LD_{50}$ |
|-----------|----------------------|-----------|
| Control | - | $1.5 \times 10^2$ |
| FDD7.7 | 0.02 | $1.5 \times 10^4$ |
| FDD7.7 | 0.2 | $4.7 \times 10^5$ |
| FDD7.7 | 2.0 | $4.7 \times 10^5$ |
| RM-5 | 10 | $3 \times 10$ |

These results show that MAb-FDD7.7 protects mice from challenge with P. aeruginosa immunotype 4 and MAb-FDD7.7 was protective at a dose of 0.02 mg per mouse. Monoclonal antibody raised against P.aeruginosa RM5 did not protect against challenge with immunotype 4 cells.

A second mouse system, CF1 in which the animals are less sensitive to P. aeruginosa was also used to evaluate the efficacy of MAb-RM5. The mice were made neutropenic with cyclophosphamide, 250 mg per kg body weight, 4 days prior to challenge. Some animals were further sensitized to bacterial challenge by the administration of 0.02 mg Fe/Kg body weight in the form of ferric ammonium citrate following the procedure of Forsberg and Bullen, J. Clin. Path. 25: 65-68 (1972), at the time of challenge. The experimental mice were treated with MAb-RM5 50 µg per mouse 2 hours prior to and 2 hours after challenge and controls with RPMI-1640. The following results were obtained.

## PROTECTION OF CF1 MICE AGAINST
## P. AERUGINOSA TYPE 2 INFECTION

| Cyclo-phosphamide | Ferric Ammonium Citrate | Treatment | $LD_{50}$ |
|---|---|---|---|
| + | − | RPMI | $2.45 \times 10^5$ |
| + | − | RM5 | $\geq 2.60 \times 10^6$ |
| + | + | RPMI | $\leq 7.93 \times 10^3$ |
| + | + | RM5 | $1.33 \times 10^5$ |

These results show that the administration of MAb-RM5 to CF1 mice that were sensitized to Pseudomonas infection with cyclophosphamide and ferric ammonium citrate raised with $LD_{50}$ from $\leq 7.93 \times 10^3$ to $1.3 \times 10^5$. MAb-RM5 raised the $LD_{50}$ from $2.45 \times 10^5$ to $2.6 \times 10^6$ in mice that were sensitized with cyclophosphamide alone.

MAb-9H10 was effective, when given I.V. 2 hours prior to challenge, in protecting Xid mice against challenge with P. aeruginosa immunotypes 3 and 6. The following results were obtained.

## PROTECTION WITH MAb-9H10

| Treatment | Dosage ug | Challenge Immunotype | LD50 |
|---|---|---|---|
| Control | ----- | 3 | $2.9 \times 10^1$ |
| 9H10 | 2.00 | 3 | $1.4 \times 10^3$ |
| 9H10 | 0.50 | 3 | $2.9 \times 10^3$ |
| 9H10 | 0.10 | 3 | $6.3 \times 10^2$ |
| Control | ----- | 6 | $2.4 \times 10^0$ |
| 9H10 | 2.00 | 6 | $1.1 \times 10^3$ |
| 9H10 | 0.50 | 6 | $4.2 \times 10^2$ |
| 9H10 | 0.10 | 6 | $6.2 \times 10^1$ |

These results show that MAb-9H10 protects mice from challenge with P. aeruginosa immunotype 5 and was protective at a dose range from 0.5 ug to 2.0 ug.

The monoclonal antibodies secreted by cell lines RM5 and FDD7.7 were tested for their ability to protect mice when administered after bacterial challenge. Immune deficient Xid mice were given antibody i.v. at different times relative to bacterial challenge.

## THERAPY OF Xid MICE INFECTED PRIOR
## TO MONOCLONAL ANTIBODY TREATMENT

| Antibody | Time Hours | µg MAb per mouse | Immunotype of Chall. Organism | $LD_{50}$ |
|---|---|---|---|---|
| MAb-RM5 | -2 | 0 | 2 | 1.0 |
| | -2 | 1 | | $1.4 \times 10^5$ |
| | -2 | 12.5 | | $1.4 \times 10^5$ |
| | 0 | 1 | | $6.7 \times 10^3$ |
| | +2 | 1 | | $3.1 \times 10^2$ |
| | +2 | 12.5 | | $4.0 \times 10^3$ |
| MAb-FDD7.7 | -1/2 | 0 | 4 | $1.0 \times 10^2$ |
| | -1/2 | 15 | | $2.7 \times 10^6$ |
| | +2 | 15 | | $3.2 \times 10^3$ |
| | +4 | 15 | | $2.5 \times 10^3$ |
| | +6 | 15 | | $1.0 \times 10^3$ |
| | +8 | 15 | | $4.7 \times 10^3$ |
| | +10 | 15 | | $2.2 \times 10^2$ |

The results show the therapeutic potential of MAb-RM5 and MAb-FDD7.7 since they protect mice when given after bacterial challenge.

## EXAMPLE 4

### GENERATION OF HYBRIDOMAS

Epstein-Barr virus transformed lymphocytes from Example 1 are fused with immortalizing cells to produce hybridoma cell lines which will continue to grow indefinitely while secreting P. aeruginosa LPS specific monoclonal antibodies. The antibody producing cells are fused with either the lymphoblastoid KR-4 cell line described by Kozbor, et al ., J. Immunol. 133: 3001-3005 or the immortalized SHM-D33 cell line described in U.S. Patent No. 4,582,797. Individual cultures of RM5, FDD7.7, 11F9 or the CF-9H10 cell line, from Example 1, $1 \times 10^7$ cells, are mixed with an equal number of KR-4 or SHM-D33 cells. The cells are washed several times with fresh RPMI-1640 and gently resuspended in 45% polyethylene glycol (PEG), mol. wt. 4000, in RPMI-1640 over a 1 minute period. The suspension is diluted 1:10 with fresh culture media over a 10 minute period and then incubated at 37°C for 30 minutes. The PEG was removed by three washings and the cell mixture plated in 0.1 ml serum supplemented culture media in flat bottom 96 well plates. Each well receives $1 \times 10^5$ cells and $5 \times 10^4$ irradiated human cord blood lymphocytes. The cells are cultured at 37°C. After 24 hours the culture medium is replaced with RPMI-1640 containing hypoxanthine ($10^{-4}$M), aminopterin ($4 \times 10^{-7}$-M), thymidine ($1.6 \times 10^{-5}$M) and oubain ($5 \times 10^{-4}$M). The culture medium was changed every 3 to 4 days and after 3 weeks, the culture fluids were assayed for the presence of antibodies reactive with Fisher, P. aeruginosa immunotype 1, 2, 3, 4, 5, 6, 7 as described in Example 1.

Out of 1,100 wells plated with the fusion product of RM5 × KR-4, two cultures were identified which produced MAb reactive with P. aeruginosa immunotype 2. The most active hybridoma has been designated RK-52 and the monoclonal antibody has been designated MAb-RK-52. The culture has been maintained for over 4 months and continues to secrete P. aeruginosa immunotype 2 specific antibody. A sample of hybridoma RK-52 was deposited in the American Type Culture Collection, 12301 Parklawn Drive, Rockville, Maryland 20852, USA, on July 24, 1986 under The Budapest Treaty and has been assigned ATCC number HB 9149.

The evaluation of 1,200 wells containing FDD7.7 × KR-4 fused cells resulted in three cultures which produce monoclonal antibody reactive with P.aeruginosa, Fisher, immunotype 4. The most active culture has continued to secrete specific monoclonal antibody for more than 5 months. The hybridoma has been designated FK-16-4 and the monoclonal antibody has been designated MAb-FK-16-4. A sample of hybridoma FK-16-4 was deposited in the American Type Culture Collection, 12301 Parklawn Drive, Rockville, Maryland 20852, USA, on July 24, 1986, under The Budapest Treaty and has been assigned ATCC number HB 9150.

The evaluation of a similar number of wells containing RM5 × SHM-D33 fused cells resulted in at least 4 cultures which produce monoclonal antibody reactive with P. aeruginosa, Fisher, immunotype 2. The most active cultures secrete at least 10 times more antibody than the parent RM5 culture. The two highest hybridomas producing the highest levels of monoclonal antibody have been designated RS-1H7 and RS-1G11 and the monoclonal antibody has been designated MAb-RS-1H7-RM5 and MAb-RS-1G11-RM5 respectively. A sample of the hybridoma RS-1H7 was deposited in the American Type Culture Collection, 12301 Parklawn Drive, Rockville, Maryland 20852, USA, on July 24, 1986, under The Budapest Treaty and has been assigned ATCC number HB9151. A sample of the hybridoma RS-1G11 was also deposited in the American Type Culture Collection, on July 1, 1987, under The Budapest Treaty and has been assigned ATCC number HB 9465.

An evaluation of more than 500 wells containing CF-9H10 × KR4 fused cells resulted in at least 2 cultures which produce monoclonal antibody following cloning by limited dilution according to Kozbor, et al. J. Immunol. 133: 3001-3005 (1984). The most active culture secretes MAb reactive with P. aeruginosa, Fisher immunotypes 3,6 and 7. The hybridoma has been designated CK-1G11-18 and the monoclonal antibody has been designated MAb-CK-1G11-18. A sample of the hybridoma CK-1G11-18 was deposited in the American Type Culture Collection, 12301 Parklawn Drive, Rockville, Maryland 20852, USA, on July 31, 1986 under The Budapest Treaty and has been assigned ATCC number HB 9161.

Isotypes of the monoclonal antibodies produced by the individual hybridomas were similar to those of the original lymphoblastoid cells prior to fusion. Immunoglobulins secreted by those cell lines were investigated as described by Kozbor et al.,J. Immunol. 133: 3001-3005 (1984). Cells were incubated in the presence of [$^{35}$S]-methionine and supernatants were analyzed by immunoprecipitation using antibodies against $\mu$ heavy chain and $x$ and $\lambda$ light chain and protein A containing Staphylococcus aureus followed by analysis of the precipitated material on polyacrylamide gels. These experiments established that cells of the KR-4 line secrete immunoglobulin and hybridomas derived from these cells, RF-52 and FK-16-4, also secrete this immunoglobulin in addition to the monoclonal antibody specific for P. aeruginosa. Supernatant fluids from RS-1H7 cells that were derived from the SHM-D33 fusion partner contained only detectable amounts of P. aeruginosa specific antibodies.

Monoclonal antibodies whether derived from lymphoblastoid cell lines or hybridomas are produced in cell culture environments and secreted into the culture fluid. The hybridoma RS-1G11 as an example was cloned at 5, 1, 1, and 1 cell per well and has been in continuous culture for 15 months while secreting more than 40 $\mu$g antibody per ml at saturating cell densities of $1 \times 10^6$ cells per ml. The twice cloned line was adapted for growth in serum free medium (HB 104) and subsequently grown in a hollow fiber system (Endotronics, Inc.). Two cartridges in an Acusyst-P system (Endotronics) were loaded with cells and 14 days later released cellular material in the culture media was collected at a flow rate of 8 ml per hour. Over a 60 day harvest period approximately 10 liters of supernatant fluid was collected containing a total of more than 3 grams of MAb-RS-1G11-RM5.


EXAMPLE 5

PASSIVE PROTECTION WITH MONOCLONAL ANTIBODIES DERIVED FROM HYBRIDOMAS

The monoclonal antibodies from Example 4 were assayed for their ability to protect mice against challenge with viable P. aeruginosa. Immune deficient Xid mice as described in Example 3 were given antibody by the intravenous route at 2 hours prior to challenge.

## PROTECTION WITH HYBRIDOMA PRODUCED MONOCLONAL ANTIBODY

| Treatment | Dose µg | Challenge Immunotype | $LD_{50}$ |
|---|---|---|---|
| Control | ----- | 2 | $3.2 \times 10^1$ |
| RK-52 | 2.00 | 2 | $3.4 \times 10^4$ |
| Control | ----- | 2 | $3.1 \times 10^1$ |
| RS-1H7 | 1.20 | 2 | $4.2 \times 10^5$ |
| RM5 | 1.20 | 2 | $3.1 \times 10^5$ |
| Control | ----- | 4 | $1.5 \times 10^2$ |
| FK-16-4 | 2.00 | 4 | $3.1 \times 10^5$ |
| FK-16-4 | 0.20 | 4 | $2.7 \times 10^5$ |
| FK-16-4 | 0.02 | 4 | $5.5 \times 10^4$ |
| FDD7.7 | 2.00 | 4 | $4.7 \times 10^5$ |
| FDD7.7 | 0.20 | 4 | $4.7 \times 10^5$ |

These results show that hybridoma produced monoclonal antibody protects mice from P. aeruginosa challenge. Antibodies produced by the RK-52, RS-1H7 and the FK-16-4 cell lines are also specific for their corresponding immunotype.

The monoclonal antibody produced by the hybridoma RS-1G11 enhanced the survival of Xid mice challenged with 500 viable P. aeruginosa immunotype 2 bacteria. Control mice receiving no MAb-RS-1G11 had a survival rate of 10%. Mice treated with 0.001 µg of MAb-RS-1G11 showed a survival rate of 70% while all of the mice treated with 0.01 µg of the antibody survived. These results demonstrate that MAb-RS-1G11 is very effective in protecting mice against Pseudomonas infection.

**Claims**

1. Human monoclonal antibodies reactive with P. aeruginosa lipopolysaccharide immunotypes and serotypes.

2. Human monoclonal antibodies according to Claim 1 wherein the antibodies react with P. aeruginosa Fisher immunotypes 2 through 7.

3. Human monoclonal antibodies according to Claim 1 wherein the antibodies react with P. aeruginosa Fisher International Antigenic Typing Systems serotypes 3 and 15.

4. A human B lymphoblastoid cell line RM-5 and the derivatives thereof.

5. A human B lymphoblastoid cell line FDD7.7 and the derivatives thereof.

6. A human B lymphoblastoid cell line CF-9H10 and the derivatives thereof.

7. The human B lymphoblastoid cell line of Claim 6 wherein the ATCC number is CRL 9477.

8. A human B lymphoblastoid cell line 11F9 and the derivatives thereof.

9. The human B lymphoblastoid cell line of Claim 8 wherein the ATCC number HB9464.

10. A human monoclonal antibody produced by the cell line of Claim 4 which reacts with P. aeruginosa Fisher immunotype 2 and has an IgM isotype.

11. A human monoclonal antibody produced by the cell line of Claim 5 which reacts with P. aeruginosa Fisher immunotype 4 and has an IgM isotype.

12. A human monoclonal antibody produced by the cell line of Claim 6 which reacts with P. aeruginosa Fisher immunotypes 3, 6 and 7 and has an IgM isotype.

13. A human monoclonal antibody produced by the cell line of Claim 8 which reacts with P. aeruginosa Fisher immunotype 5 and has an IgM isotype.

14. A human monoclonal antibody designated MAb-RM5.

15. A human monoclonal antibody designated MAb-FDD7.7.

16. A human monoclonal antibody designated MAb-9H10.

17. A human monoclonal antibody designated MAb-11F9.

18. A hybridoma produced from the fusion of the lymphoblastoid cell line of Claim 4 and an immortalizing cell and the derivatives thereof.

19. A hybridoma of Claim 18 designated RK-52 and having the ATCC number HB 9149.

20. A hybridoma of Claim 18 designated RS-1H7 and having the ATCC number HB 9151.

21. A hybridoma of Claim 18 designated RS-1G11 and having the ATCC number HB 9465.

22. A hybridoma produced from the fusion of the lymphoblastoid cell line of Claim 5 and an immortalizing cell and the derivatives thereof.

23. A hybridoma of Claim 22 designated FK-16-4 and having the ATCC number HB 9150.

24. The hybridoma produced from the fusion of the lymphoblastoid cell line of Claim 6 and an immortalizing cell and the derivatives thereof.

25. A hybridoma of Claim 24 designated CK-1G11-18 and having the ATCC number HB 9161.

26. A hybridoma produced from the fusion of the lymphoblastoid cell line of Claim 8 and an immortalizing cell and the derivatives thereof.

27. Human monoclonal antibody produced by the hybridoma of any one of Claims 18, 22, 24 and 26.

28. A pharmaceutical composition for treating or preventing P. aeruginosa infection comprising an effective amount of monoclonal antibodies reactive with P. aeruginosa Fisher immunotypes in a pharmaceutically acceptable carrier.

29. The use of monoclonal antibodies reactive with P. aeruginosa Fisher immunotypes for the preparation of a medicament useful for treating or preventing P. aeruginosa infections.

30. A method of identifying P. aeruginosa Fisher immunotypes 2 through 7 and International Antigenic Typing System serotypes 3 and 15 comprising the contacting of P. aeruginosa cells or cell free lipopolysaccharide with human monoclonal antibodies specifically reactive with immunotypes 2 through 7 and serotypes 3 and 15 and determining antibody binding.

31. A pharmaceutical composition for treating or preventing P. aeruginosa infection comprising an effective amount of monoclonal antibodies reactive with P. aeruginosa Fisher immunotypes administered with an effective amount of an antibiotic selected from the antibiotic classes consisting of aminoglycoside, cephalosporin, macrolid, penicillin, tetracycline, polypeptide and polyene in a pharmaceutically acceptable carrier.